# EUROPEAN PATENT SPECIFICATION

(11) **EP 2 924 125 B1**
(45) Date of publication and mention of the grant of the patent: **14.06.2017**
(21) Application number: 14161897.5
(22) Date of filing: 27.03.2014
(51) Int. Cl.: C12Q 1/68

(54) **Method of predicting the tumor response to DNA methylation inhibitors and alternative therapeutic regimens for overcoming resistance**
Verfahren zur Vorhersage der Tumorreaktion auf Inhibitoren der DNA-Methylierung und alternative Therapieregime zur Überwindung von Resistenz
Procédé de prédiction de la réponse tumorale à des inhibiteurs de la méthylation d'ADN et autres régimes thérapeutiques pour surmonter la résistance

(43) Date of publication of application: 30.09.2015
(60) Divisional application: 16158309.1
(73) Proprietor: Palacky University, Olomouc, 77147 Olomouc (CZ)
(72) Inventor: Agrawal, Khushboo, 77900 Olomouc (CZ); Dzubak, Petr, 75103 Brodek u Prerova (CZ); Hajduch, Marian, 79305 Moravsky Beroun (CZ); Frydrych, Ivo, 75114 Drevohostice (CZ)
(74) Representative: Hartvichova, Katerina

(56) References cited:
- WO-A1-2014/025582
- US-A1- 2011 301 110
- ROMAIN AUCAGNE ET AL: "Transcription intermediary factor 1 gamma is a tumor suppressor in mouse and human chronic myelomonocytic leukemia", JOURNAL OF CLINICAL INVESTIGATION, AMERICAN SOCIETY FOR CLINICAL INVESTIGATION, US, vol. 121, no. 6, 1 June 2011 (2011-06-01), pages 2361-2370, XP002684885, ISSN: 0021-9738, DOI: 10.1172/JCI45213 [retrieved on 2011-05-02]
- T. BRAUN ET AL: "Molecular predictors of response to decitabine in advanced chronic myelomonocytic leukemia: a phase 2 trial", BLOOD, vol. 118, no. 14, 6 October 2011 (2011-10-06), pages 3824-3831, XP055059701, ISSN: 0006-4971, DOI: 10.1182/blood-2011-05-352039
- S BOHL ET AL: "Decitabine Response Associated Gene Expression Patterns In Acute Myeloid Leukemia (AML)", 55TH ASH ANNUAL MEETING AND EXPOSITION, 9 December 2013 (2013-12-09), XP055128766,
- ABEL GONZALEZ-PEREZ ET AL: "The mutational landscape of chromatin regulatory factors across 4,623 tumor samples", GENOME BIOLOGY, BIOMED CENTRAL LTD., LONDON, GB, vol. 14, no. 9, 24 September 2013 (2013-09-24), page r106, XP021169150, ISSN: 1465-6906, DOI: 10.1186/GB-2013-14-9-R106
- BRITTA SKAWRAN ET AL: "Gene expression profiling in hepatocellular carcinoma: upregulation of genes in amplified chromosome regions", MODERN PATHOLOGY, vol. 21, no. 5, 15 February 2008 (2008-02-15), pages 505-516, XP055120748, ISSN: 0893-3952, DOI: 10.1038/modpathol.3800998
- PANAGIS FILIPPAKOPOULOS ET AL: "The bromodomain interaction module", FEBS LETTERS, vol. 586, no. 17, 1 August 2012 (2012-08-01), pages 2692-2704, XP055128985, ISSN: 0014-5793, DOI: 10.1016/j.febslet.2012.04.045

## Description

### Field of Art

The invention is directed to a method for predicting the tumor response (i.e. sensitive or resistant) towards DNA methylation inhibitors as well as it provides alternative therapeutic regimens to overcome the resistance.

### Background Art

Resistance to chemotherapeutic treatment is one of the major impediments forefending the successful cancer therapy (Gottesman M.M. et al., Nature Reviews Cancer 2002; 2, 48-58). Although the research has unraveled the main molecular signatures of resistance to chemotherapy, including intracellular inactivation of the drug (Garattini S. et al., European Journal of Cancer 2007; 43, 271-82), defects in DNA mismatch repair (Fink D. et al., Clinical Cancer Research 1998; 4, 1-6), evasion of apoptosis (Hanahan D. et al., Cell 2000; 100, 57-70), membrane transporters (Huang Y. et al., Cancer Research 2004; 64, 4294-301) and many more, the failure of cancer chemotherapy remains frequently unresolved. Moreover, a particular drug resistance mechanism defined in cell culture systems and animal models does not necessarily correlate with the individual molecular pathology in clinic (Cimoli G. et al., Biochimica et Biophysica Acta 2004; 1705, 103-20). This has underlined the paramount importance for investigating the additional targets to sensitize the cancer patients, resistant to a particular drug, and tailor an alternative therapeutic regimen for the individual patients. Currently, epigenetics has emerged as one of the most promising fields expanding the boundaries of oncology and aberrant DNA methylation remains the consistent hallmark due to its frequent involvement in all types of cancer (Rodríguez-Paredes M. et al., Nature Medicine 2011; 17, 330-39). Cytosine analogues, 5-azacytidine (azacytidine) and 2'-deoxy-5-azacytidine (decitabine) are currently one of the most effective epigenetic drugs (Stresemann C. et al., International Journal of Cancer 2008; 123, 8-13), which function by inhibiting the expression of *de novo* DNA methyltransferases and have shown substantial potency in reactivating tumor suppressor genes silenced by aberrant DNA methylation (Karahoca M. et al., Clinical Epigenetics 2013; 5, 3). The prototypical DNA methyltransferase inhibitors, azacytidine and decitabine are one of the few drugs that patients suffering from myelodysplastic syndromes (MDS) and acute myeloid leukemia (AML) respond to, and have been approved by the Food And Drug Administration (FDA) and European Medicines Agency (EMA) for the treatment of MDS (Saba H.I. et al., Therapeutics and Clinical Risk Management 2007; 3, 807-17). Apart from being established therapies for myeloid malignancies, they seemed promising in eradicating solid tumors during early clinical trials (Cowan L.A. et al., Epigenomics 2010; 2, 71-86). However, like other anti-cancer drugs, resistance to these hypomethylating agents is the major barrier, reversing the effective epigenetic therapy. Most patients do not respond to therapy and experience primary resistance whereas those responding initially acquire secondary resistance and succumb to the disease, despite continued therapy (Prébet T. et al., Journal of Clinical Oncology 2011; 29, 3322-7). Molecular mechanisms dilating the cause of resistance to these drugs *in vitro* are diverse, including insufficient drug influx by membrane transporters, deficiency of the enzyme deoxycytidine kinase required for drug activation, or deamination by cytidine deaminase leading to increased drug metabolism, but they fail to explain acquired resistance in patients. In addition, it has also been implemented that the secondary resistance to decitabine is likely to be independent of DNA methylation and the resistance develops regardless of persistent demethylation (Qin T. et al., PLOS ONE 2011; 6, e23372). Also, it is undeniable fact that the re-expression of epigenetically silenced tumor suppressor genes following decitabine treatment is transitory (Kagey J.D. et al., Molecular Cancer Research 2010; 8, 1048-59). Withdrawal of decitabine eventually results in gene re-silencing leading to resistance whereas sustained gene re-expression concords with the clinical response, supporting the role of gene re-silencing in development of drug resistance (Hesson L.B. et al., PLOS Genetics 2013; 9, e1003636). If the focus is laid on gene re-silencing as the prerequisite for resistance, it highlights the central dogma of epigenetics which articulates that the gene silencing mechanisms (DNA hypermethylation, mutations in chromatin remodeling complexes and multiple post-translational histone modifications) are not isolated from each other but interlinked (Grant S. et al., Clinical Cancer Research 2009; 15, 7111-3). In this context, bromodomains (BRDs), chromatin effector modules that recognize and bind to ε-N-acetyl lysine motifs have rapidly emerged as exciting new targets in the quest for clinical progress in cancer (Muller S. et al., Expert Reviews in Molecular Medicine). The role of multiple bromodomain genes in restricting the spread of heterochromatic silencing has been explored in the past (Jambunathan N. et al., Genetics 2005; 171, 913-22). In addition, bromodomain proteins play a critical role in gene activation by recruitment of the factors necessary for transcription (Josling G.A. et al., Genes 2012; 3, 320-43). Human BRD family comprises 47 bromodomain containing genes and/or proteins including the bromodomain and extra terminal domain (BET) family composed of BRD2, BRD3, BRD4, and BRDT (Puissant A. et al., Cancer Discovery 2012; 3, 1-16). Braun et al. (Blood 2008; 118, 3824-3831), discloses expression markers of response to decitabine, a well known type of DNA methylation inhibitor, in advanced chronic myelomonocytic leukemia. The present invention exposes such bromodomain containing genes and/or proteins coded by the gene, the expression of which was differentially regulated during the development of resistance, and targeting of which may sensitize the patients suffering from resistance towards DNA methylation inhibitors. Therefore, the present invention provides a method for determining the response of the patients (i.e. sensitive or resistant) towards DNA methylation inhibitors and also provides the alternative therapeutic regimens to resolve the resistance.

### Disclosure of the invention

The first embodiment of the invention is a method for predicting the sensitivity of a patient suffering from a cancer disease to DNA methylation inhibitor therapy, which comprises determining *in vitro* in the cancer cells taken from the patient and comparing with values for parent type of cells
- the level of expression of bromodomain containing gene ASH1L, wherein the change in expression determining resistance is increase,
and/or
- the mutations involving the non-synonymous change in amino acid sequence of bromodomain containing protein ASH1L:

| | **Amino acid change determining resistance** | | **Mutation in parental vs resistant cell lines** | |
|---|---|---|---|---|
| | **Position** | **Reference** | **Parental** | **Resistant** |
| ASH1L | 1429 | Alanine | Alanine | Valine |

and subsequently determining the resistance or sensitivity of the patient towards the said treatment based on the information provided above.

In a preferred embodiment, the method further comprises determining *in vitro* in cancer cells taken from the patient and comparing with values for parent type of cells
- the level of expression of at least one further bromodomain containing gene selected from the group comprising:

| **Gene** | **Change in expression determining resistance** |
|---|---|
| ATAD2 | decrease |
| BAZ1B | decrease |
| BAZ2A | increase |
| BAZ2B | decrease |
| BRD1 | decrease |
| BRD3 | decrease |
| BRD7 | decrease |
| BRD8 | decrease |
| BRWD1 | increase |
| CECR2 | increase |
| CREBBP | increase |
| EP300 | increase |
| KAT2A | increase |
| KAT2B | increase |
| KMT2A | increase |
| SMARCA2 | increase |
| SP100 | increase |
| SP110 | increase |
| TRIM66 | decrease |
| ZMYND8 | decrease |
| ZMYND11 | decrease |

and/or
- the level of expression of at least one bromodomain containing protein selected from the group comprising:

| **Protein** | **Change in expression determining resistance** |
|---|---|
| ATPase family, AAA domain containing 2 | decrease |
| bromodomain adjacent to zinc finger domain, 1A | decrease |
| bromodomain adjacent to zinc finger domain, 1B | increase |
| bromodomain adjacent to zinc finger domain, 2A | decrease |
| bromodomain PHD finger transcription factor | increase |
| bromodomain containing 2 | decrease |
| bromodomain containing 8 | increase |
| cat eye syndrome chromosome region, candidate 2 | increase |
| CREB binding protein | decrease |
| lysine (K)-specific methyltransferase 2A | increase |
| polybromo 1 | increase |
| pleckstrin homology domain interacting protein | increase |
| SWI/SNF related, matrix associated, actin dependent regulator of chromatin, subfamily a, member 4 | increase |
| SP100 nuclear antigen | increase |
| TAF1 RNA polymerase II, TATA box binding protein (TBP)-associated factor, 250kDa | increase |
| tripartite motif containing 28 | decrease |
| tripartite motif containing 33 | increase |

and/or
- the mutations involving the non-synonymous change in amino acid sequence of at least one further bromodomain containing protein selected from the group comprising:

| | **Amino acid change determining resistance** | | **Mutation in parental vs resistant cell lines** | |
|---|---|---|---|---|
| | **Position** | **Reference** | **Parental** | **Resistant** |
| ATAD2 | 365 | Serine | Serine | Phenylalanine |
| ATAD2B | 207 | Glutamine | Arginine | Glutamine |
| BAZ2A | 1 | Methionine | Isoleucine | Methionine |
| BAZ2A | 650 | Glycine | Glycine | Alanine |
| KAT2A | 781 | Arginine | Arginine | Proline |
| SMARCA2 | 855 | Arginine | Glutamine | Arginine |
| TRIM24 | 478 | Proline | Leucine | Proline |
| TRIM24 | 512 | Proline | Leucine | Proline |
| TRIM33 | 286 | Leucine | Leucine | Proline |
| TRIM66 | 630 | Leucine | Valine | Leucine |
| TRIM66 | 324 | Histidine | Arginine | Histidine |
| TRIM66 | 466 | Histidine | Histidine | Arginine |

The change in the level of expression (up-regulation or down-regulation) of the genes and/or proteins coded by the genes, listed in the relevant table was observed repeatedly in several drug resistant cell lines in comparison with their genetically identical drug sensitive counterpart, and is therefore the indicator of resistance towards DNA methylation inhibitor, 2'-deoxy-5-azacytidine (DAC). Preferably, the level of expression of a combination of at least two, three, four, five, six, seven, eight, nine or ten bromodomain containing genes and optionally also the level of expression of a combination of at least two, three, four, five, six, seven, eight, nine or ten bromodomain containing proteins is determined. Most preferably, the level of expression of all the listed bromodomain containing genes and optionally also the level of expression of all the listed bromodomain containing proteins is determined.

The mutations in bromodomain containing genes at given reference position was observed repeatedly between the drug resistant cell lines and their genetically identical drug sensitive counterpart in comparison with the human reference genome, and is therefore the indicator of resistance towards DNA methylation inhibitor, DAC. Preferably, the mutations at given reference position in combination of at least two, three, four, five, six, seven, eight, nine or ten bromodomain containing genes is determined. Most preferably, the mutations in all the listed bromodomain containing genes are determined.

The increase in the IC₅₀ values of the tested epigenetic inhibitors was determined repeatedly in several drug resistant cell lines in comparison with their genetically identical drug sensitive counterpart, which indicates towards the cross-resistance of the DAC resistant cells, to other epigenetic inhibitors. Therefore, the gene and protein expression data mentioned in the present discovery can also be applied for predicting the sensitivity and/or resistance towards other epigenetic drugs with mode of actions similar to 2'-deoxy-5-azacytidine.

The method of determination of resistance and the combination therapy are particularly useful in cancers selected from carcinomas, sarcomas, melanomas, lymphomas, and leukemia.

### Examples of currying out the invention

### Cell culture/Development of resistant cell lines

We used the cell line derived from the patient suffering from human colorectal cancer, HCT116 (American Type Culture Collection, Manassas, VA, USA) to study the mechanism of resistance towards DNA methylation inhibitor, 2'-deoxy-5-azacytidine. Cell line was maintained in McCoy's 5A medium (Sigma-Aldrich, St. Louis, MO) supplemented with 10% fetal bovine serum (PAN-Biotech GmbH, Aidenbach, Germany), 100 U/mL penicillin (Biotika, Slovenská L'up 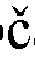 a, Slovak Republic), 50 µg/mL streptomycin and 3 mM L-glutamine (Sigma-Aldrich) at 37°C and 5% CO₂.

The resistant cell lines were developed using two methods. For the purpose, early passage HCT116 cells were split and seeded into two petridishes. The cells in Petridish A were initially treated with 1x IC₅₀ concentration of the drug (0.28 µM) which was gradually increased with the adaptation of resistance up to 10x IC₅₀ in subsequent passages. The cells in Petridish B were directly exposed to 5x IC₅₀ concentration of the drug which was further doubled to 10x IC₅₀. After long term exposure of the cells to cytotoxic dose of the drug, the bulk population was determined to be resistant. Cloning of the resistant cell population resulted in six resistant cell lines, R1.1, R1.2, R1.3, R1.4 (isolated from Petridish A) and R2.1, R2.2 (isolated from Petridish B).

Resistance was confirmed by the MTT-based cell survival assay and the resistance index was calculated as the fold increase in the IC₅₀ of the resistant cell lines compared with the untreated control. All of the resistant cell lines were > 100 folds resistant to DAC.

### RNA sequencing (RNA-Seq) based transcriptomics

RNA sequencing (RNA-Seq) utilizing high-throughput sequencing platforms have emerged as the powerful method for discovering, profiling and quantifying transcriptome by facilitating relatively unbiased measurements of transcript and gene expressions, ability to measure exon and allele specific expressions and to detect the transcription of unannotated exons leading to the identification of rare and novel transcripts (Pickrell J.K. et al., Nature 2010; 464, 768-72).

### Sample preparation/construction of cDNA library:

HCT116 parental and each of the six resistant cell lines were grown in petridishes with coverage greater than 80%. Cells were homogenized using 1 mL of TRI (trizol) reagent per 10 cm² of the monolayer culture and incubated at room temperature for 5 min to allow the complete dissociation of the nucleoprotein complexes after which the homogenates were transferred to 1.5 mL microcentrifuge tubes and the total RNA was extracted by the organic extraction method according to the manufacturer's protocol (Ambion RiboPure Kit, Life Technologies, Carlsbad, CA). The integrity of the obtained RNA samples was analyzed (Agilent RNA 6000 Nano Kit, Agilent Technologies, Santa Clara, CA) using Agilent 2100 Bioanalyzer.

0.1-4 µg of the total RNA was then used and the cDNA library was constructed according to the manufacturer's protocol (TruSeq Stranded mRNA Sample Prep Kit, Illumina, San Diego, CA). Briefly, the poly-A containing mRNA molecules were purified using poly-T oligo attached magnetic beads in two rounds of purification which included RNA fragmentation and priming with random hexamers. The cleaved RNA fragments were then reverse transcribed (RevertAid H Minus Reverse Transcriptase, Thermo Scientific, Waltham, MA) into first strand cDNA followed by second strand cDNA synthesis. The cDNA synthesis was complemented with an "End Repair" control at -20°C. A single 'A' nucleotide was then added to the 3' ends of the blunt fragments followed by the ligation of multiple indexing adaptors. The DNA fragments with adapter molecules on both ends were then selectively enriched and amplified by PCR.

The cDNA library thus prepared was validated and quantified (Agilent High Sensitivity DNA Kit, Agilent Technologies) using Agilent 2100 Bioanalyzer. Finally, the samples with different indexes were pooled together for sequencing.

### RNA Sequenceing, Alignment and Valiant Calling:

Transcriptome was sequenced by massively parallel signature sequencing (MPSS) using the Illumina's ultra-high-throughput sequencing system, HiSeq 2500.

The reads generated from the RNA Seq experiment were aligned to annotated human reference genome (HG₁₉) using Tophat 2 (Trapnell C. et al., Bioinformatics 2009; 25, 1105-11) and those aligning to exons, genes and splice junctions were counted. Tolerance was set to allow the maximum of two mismatches during an alignment and the reads aligning to multiple genomic locations were discounted.

Variants (cSNPs, indels and splice junctions) were called after alignment by SAMtools (Li H. et al., Bioinformatics 2009; 25, 2078-79) and annotated by ANNOVAR (Wang K. et al., Nucleic Acids Research 2010; 38, e164).

For the quantification of gene and transcript level expression, HTSeq package (Python) was used and the differential expressions were reported after data normalization and statistical evaluation using DESeq package (R library). Statistical significance was determined by the binomial test and the threshold for significance was set to 0.01.

### Mass spectrometry based proteomics

While transcriptomics studies provide insight into the roles of RNA and gene expression, it is ultimately the change in the level of protein expressions which affects the biological functions. Stable isotope labelling of amino acids in cell culture (SILAC) coupled with mass spectrometry has evolved as an invaluable tool in identification and development of novel biomarkers by facilitating the quantification of differential protein levels in normal and pathophysiological states (Mann M., Nature Reviews Molecular Cell Biology 2006; 7, 952-58).

### SILAC/Preparation of lysates:

The parental cell line, HCT116 was cultured in SILAC medium (Thermo Scientific) substituted with heavy Lys-¹³C₆ and Arg-¹³C₆ and dialyzed fetal bovine serum (Sigma-Aldrich) for about 8 doublings to reach the complete labelling. The labelled parental cell line was then mixed with each of the non-labeled resistant cell lines in 1:1 ratio. Cell mixture thus prepared was washed twice with ice cold 1x PBS with inhibitors [phosphatase inhibitors (5 mM sodium pyrophosphate, 1 mM sodium orthovanadate, 5 mM sodium fluoride), protease inhibitors (1 mM phenylmethylsulfonyl fluoride, Protease Inhibitor Cocktail; Sigma-Aldrich)] followed by a wash with 1x PBS without inhibitors after which the cells were lysed using 200 µL of ice cold SILAC lysis buffer [20 mM Tris-HCl, 7 M Urea, 10 mM DTT (dithiothreitol), 1% Triton X-100, 0.5% SDS] per 2 x 10⁷ cells. Lysates were then sonicated using Branson Digital Sonifier and clarified by centrifugation at 14,000 rpm for 10 min and cleared supernatants were stored at -80°C.

### Fractionation/Enzymatic in-gel digestion:

Cell lysates (100 µL) were fractionated by molecular weight on 12% LDS-Tris-Glycine gel through a cylindrical gel matrix by continuous-elution gel electrophoresis (Mini Prep Cell, Bio-Rad, Hercules, CA) at constant power of 1 W for 3-4 hours. After electrophoresis, the gel was expelled from the tube and fixed (10% acetic acid, 35% ethanol) followed by rinsing with Milli-Q H₂O. Using a clean scalpel, the 90 mm gel piece was then excised into 20 slices (∼4.5 mm each) which were further diced into small pieces (∼1 mm³) and transferred to 1.5 mL microcentrifuge tubes.

The gel pieces were then dehydrated with ACN (acetonitrile) by sonication for 5 min followed by reduction with 50 mM tris-(2-carboxyethyl)phosphine at 90°C for 10 min. The reduced gel was dehydrated again followed by alkylation with freshly prepared 50 mM IAA (iodoacetamide) for 60 min in dark. After alkylation, the gel was dehydrated twice with changes of ACN and H₂O (to ensure the complete removal of IAA) followed by dehydration with 50% ACN at last. Finally, the gel was subjected to enzymatic digestion by overnight incubation at 37°C with trypsin buffer (Trypsin Gold, Promega, Madison, WI) prepared according to manufacturer's protocol.

After in-gel digestion of proteins, the peptide mixtures were extracted by dehydrating the gel [0.1% TFA (trifluoroacetic acid), 80% ACN] followed by rehydration (0.1% TFA) and dehydration with 50% ACN at last. The extraction buffer was then concentrated until complete evaporation and the peptide mixtures were re-suspended (5 µL 80% ACN, 0.1% TFA) and diluted (145 µL 0.1% TFA). 150 µL of the peptide samples were then loaded onto the column (MacroTrap, MICHROM Bioresources Inc., Auburn, CA) and desalted (0.1% TFA) followed by elution (0.1% TFA, 80% ACN). After purification, the elution buffer was completely evaporated and the purified peptides were re-suspended in mobile phase A [5% ACN, 0.1% FA (Formic acid)] for LC-MS analysis.

### LC-MS/MS:

20 µL of peptide samples in mobile phase A were loaded onto the trap column (Acclaim PepMap100 C18, 3 µm, 100 Å, 75 µm i.d. x 2 cm, nanoViper, Thermo Scientific) in UltiMate 3000 RSLCnano system (Thermo Scientific) for pre-concentration and desalting at the flow rate of 5 µL/min. The trap column in turn was directly connected with the separation column (PepMap C18, 3 µm, 100 Å, 75 µm i.d. x 15 cm, Thermo Scientific) in EASY-Spray (Thermo Scientific) and the peptides separated by reverse-phase chromatography were eluted with 100 min linear gradient from 5 to 35% mobile phase B (80% ACN, 0.1% FA) at the flow rate of 300 nL/min and 35°C column temperature. After the gradient, the column was washed with mobile phase B and re-equilibrated with mobile phase A. For the acquisition of mass spectra, high performance liquid chromatography was coupled to an Orbitrap Elite Mass Spectrometer (Thermo Scientific) and the spectra were acquired in a data dependent manner with an automatic switch between MS and MS/MS scans using a top 20 method. MS spectra were acquired using Orbitrap analyzer with a mass range of 300-1700 Da and a target value of 10⁶ ions whereas MS/MS spectra were acquired using ion trap analyzer and a target value of 10⁴ ions. Peptide fragmentation was performed using CID method and ion selection threshold was set to 1000 counts.

### Data analysis:

Raw MS files were analyzed by MaxQuant version 1.4.1.3 (Cox J. et al., Nature Biotechnology 2008; 26, 1367-72) and MS/MS spectra was searched using Andromeda search engine (Cox J. et al., Journal of Proteome Research 2011; 10, 1794-1805) against the UniprotKB/Swiss-Prot-human database (generated from version 2013_09) containing forward and reverse sequences. The additional database of 248 common contaminants was included during the search (Geiger T. et al., Molecular & Cellular Proteomics 2012; 11, M111.014050). Mass calibration was done using the results from the initial search with a precursor mass tolerance of 20 ppm, however, in the main Andromeda search, the precursor mass and the fragment mass was set to the tolerance of 7 ppm and 20 ppm respectively. The fixed modification of carbamidomethyl cysteine and the variable modifications of methionine oxidation and N-terminal acetylation were included for database searching. SILAC labels, R6 and K6 were used for the analysis of SILAC data. The search was based on the enzymatic cleavage rule of Trypsin/P and a maximum of two miscleavages were allowed. The minimal peptide length was set to six amino acids and at least one unique peptide was must for protein identification. The false discovery rate (FDR) for the identification of peptide and protein was set to 0.01.

Bioinformatic analysis was then performed with Perseus version 1.4.1.3. Filtrations were done to eradicate the identifications from the databases of the reverse sequence and the common contaminants and to exclude proteins with < 3 valid values (only peptides quantified in three measurements were considered). The categorical annotation was supplied in the form of Gene Ontology (GO) biological process, molecular function and cellular component. For the quantification of differential expression, the data was transformed to log 2 and normalized by subtracting the median from each column. The fold change was calculated as the mean of the three values and significance was determined by calculating the p-value with a Benjamini-Hochberg multiple hypothesis testing correction based on the FDR threshold of 0.05.

### Results

The gene and protein expression studies were done at RNA and protein levels respectively. For the gene expression studies, massively parallel signature sequencing (MPSS) was used and the sequences generated from the RNA-Seq experiment were mapped on annotated human reference genome (HG₁₉) followed by quantification of gene and transcript expressions, whereas for the protein expression studies, stable isotope labelling of amino acids in cell culture (SILAC) was used and the protein expressions were quantified using mass spectrometry.

For reporting the differential expressions, each of the drug resistant cell lines, HCT116-DAC (R1.1, R1.2, R1.3, R1.4 and R2.1, R2.2) were compared with their genetically identical drug sensitive counterpart or the parental cell line, HCT116.

Values are represented as fold changes. Positive values indicate up-regulation and negative values indicate down-regulation. The data is generated from three independent experiments and is statistically significant (p-value <0.05).

| **Gene** | **Change in expression determining resistance** | **Average fold change in DAC resistant cell lines** | | | | | |
|---|---|---|---|---|---|---|---|
| | | **R1.1** | **R1.2** | **R1.3** | **R1.4** | **R2.1** | **R2.2** |
| ASH1L | increase | | | | | | 0.52 |
| ATAD2 | decrease | | | | | -0.67 | -0.64 |
| BAZ1B | decrease | | | | | -0.40 | |
| BAZ2A | increase | | | 0.33 | 0.33 | | 0.38 |
| BAZ2B | decrease | | | | -0.92 | | |
| BRD1 | decrease | -0.48 | -0.52 | | | | |
| BRD3 | decrease | | -0.37 | | | | |
| BRD7 | decrease | | | | | | -0.55 |
| BRD8 | decrease | -0.44 | -0.35 | | -0.47 | -0.67 | -0.50 |
| BRWD1 | increase | 0.66 | | | | | |
| CECR2 | increase | | | | | | 0.73 |
| CREBBP | increase | | | 0.35 | 0.40 | | |
| EP300 | increase | | | | | | 0.35 |
| KAT2A | increase | 0.37 | | 0.47 | 0.40 | 0.43 | |
| KAT2B | increase | | | | | | 0.62 |
| KMT2A | increase | | 0.45 | | | | |
| SMARCA2 | increase | 0.71 | 0.73 | 0.69 | 0.98 | 0.76 | 1.17 |
| SP100 | increase | 0.77 | 0.59 | | 0.99 | 0.51 | 1.22 |
| SP110 | increase | 0.85 | 0.58 | | 1.29 | | 1.19 |
| TRIM66 | decrease | | | -0.60 | -0.55 | | |
| ZMYND8 | decrease | | | | | -0.50 | -0.50 |
| ZMYND11 | decrease | | -0.55 | | | -0.43 | |

| **Protein** | **Change in expression determining resistance** | **Average fold change in DAC resistant cell lines** | | | | | |
|---|---|---|---|---|---|---|---|
| | | **R1.1** | **R1.2** | **R1.3** | **R1.4** | **R2.1** | **R2.2** |
| ATPase family, AAA domain containing 2 | decrease | -0.10 | -1.19 | -0.33 | -0.78 | -0.22 | - 0.31 |
| bromodomain adjacent to zinc finger domain, 1A | decrease | -0.69 | -0.58 | -0.53 | -0.68 | -0.58 | - 0.54 |
| bromodomain adjacent to zinc finger domain, 1B | increase | 0.38 | 0.27 | 0.22 | 0.31 | 0.29 | 0.15 |
| bromodomain adjacent to zinc finger domain, 2A | decrease | - 0.66 | | -0.34 | -0.47 | -0.16 | - 0.32 |
| bromodomain PHD finger transcription factor | increase | 0.07 | | -0.01 | -0.09 | 0.05 | |
| bromodomain containing 2 | decrease | -0.70 | | -0.54 | -0.32 | -0.28 | |
| bromodomain containing 8 | increase | | | | | | 1.78 |
| cat eye syndrome chromosome region, candidate 2 | increase | 0.61 | 0.54 | 0.55 | 0.44 | 0.17 | 0.03 |
| CREB binding protein | decrease | | -0.67 | -0.38 | -0.09 | -0.14 | |
| lysine (K)-specific methyltransferase 2A | increase | | 0.00 | | | 0.04 | |
| polybromo 1 | increase | 0.52 | | 0.53 | 0.26 | 0.47 | |
| pleckstrin homology domain interacting protein | increase | 0.52 | | 0.31 | | - 0.01 | |
| SWI/SNF related, matrix associated, actin dependent regulator of chromatin, subfamily a, member 4 | increase | 0.58 | 0.55 | 0.42 | 0.43 | 0.31 | 0.34 |
| SP100 nuclear antigen | increase | -0.26 | | 0.02 | -0.16 | 0.11 | 1.09 |
| TAF1 RNA polymerase II, TATA box binding protein (TBP)-associated factor, 250kDa | increase | | 0.60 | | 0.38 | 0.25 | |
| tripartite motif containing 28 | decrease | -0.03 | -0.33 | -0.22 | -0.20 | -0.09 | -0.24 |
| tripartite motif containing 33 | increase | 0.27 | | 0.23 | 0.12 | 0.29 | 0.95 |

The mutations involving non-synonymous change in amino acid sequence was determined using the data generated from the RNA Seq experiment, after the alignment step.

The validity of the mutations represented in the table is determined by the high quality and coverage of the reads (>100). Moreover, these mutations were identified at least in three independent sequencing experiments.

| **Gene** | **Amino acid change determining resistance** | | **Mutation in parental vs resistant cell lines** | |
|---|---|---|---|---|
| | **Position** | **Reference** | **HCT116** | **HCT116-DAC** |
| ASH1L | 1429 | Alanine | Alanine | Valine (R2.1) |
| ATAD2 | 365 | Serine | Serine | Phenylalanine (R1.1, R1.2, |
| | | | | R1.3) |
| ATAD2B | 207 | Glutamine | Arginine | Glutamine (R1.2) |
| BAZ2A | 1 | Methionine | Isoleucine | Methionine (R1.1, R1.2, R1.3, R1.4, R2.1) |
| BAZ2A | 650 | Glycine | Glycine | Alanine (R1.3) |
| KAT2A | 781 | Arginine | Arginine | Proline (R2.1) |
| SMARCA2 | 855 | Arginine | Glutamine | Arginine (R1.2) |
| TRIM24 | 478 | Proline | Leucine | Proline (R2.1) |
| TRIM24 | 512 | Proline | Leucine | Proline (R2.1) |
| TRIM33 | 286 | Leucine | Leucine | Proline (R2.2) |
| TRIM66 | 630 | Leucine | Valine | Leucine (R1.2, R1.3, R1.4, R2.2) |
| TRIM66 | 324 | Histidine | Arginine | Histidine (R1.2, R1.3) |
| TRIM66 | 466 | Histidine | Histidine | Arginine (R1.4) |

### Industrial applicability

Bromodomain containing genes and/or proteins disclosed in the present invention can be used as the biomarkers for predicting the clinical response towards the epigenetic therapy targeting aberrant DNA methylation. The varying level of expression of the genes and/or proteins and the mutations involving non-synonymous change in amino acid sequence can be used as a fundament to differentiate between the responders and the non-responders. This provides the accessibility of the method of prediction, and personalization of the therapy.

The patients who do not respond to the DNA methylation inhibitors and suffer from the primary resistance can be quickly eliminated from the ineffective treatment. This will provide the benefit to such patients by escape from the relative side effects that might associate with the drug, redundant cost of therapy, and suggests for other possible treatment protocol in time.

The patients who initially respond to the drug but during prolonged treatment develop the sign of disease progression by acquiring secondary resistance to the drug can be resensitized by the use of a bromodomain inhibitor in combination with a DNA methylation inhibitor. This provides the alternative therapeutic regimen to overcome the resistance and may reduce the incidence of developing resistance to a particular DNA methylation inhibitor.

## Claims

1. A method for predicting the sensitivity of a patient suffering from a cancer disease to DNA methylation inhibitor therapy, which comprises determining *in vitro* in cancer cells taken from the patient and comparing with values for parent type of cells
- the level of expression of bromodomain containing gene ASH1L, wherein the change in expression determining resistance is increase,
and/or
- the mutations involving the non-synonymous change in amino acid sequence of bromodomain containing protein ASH1L:
| | **Amino acid change determining resistance** | | **Mutation in parental vs resistant cell lines** | |
|---|---|---|---|---|
| | **Position** | **Reference** | **Parental** | **Resistant** |
| ASH1L | 1429 | Alanine | Alanine | Valine |

2. The method for predicting the sensitivity of a patient suffering from a cancer disease to DNA methylation inhibitor therapy according to claim 1, which further comprises determining *in vitro* in cancer cells taken from the patient and comparing with values for parent type of cells
- the level of expression of at least one further bromodomain containing gene selected from the group comprising:
| **Gene** | **Change in expression determining resistance** |
|---|---|
| ATAD2 | decrease |
| BAZ1B | decrease |
| BAZ2A | increase |
| BAZ2B | decrease |
| BRD1 | decrease |
| BRD3 | decrease |
| BRD7 | decrease |
| BRD8 | decrease |
| BRWD1 | increase |
| CECR2 | increase |
| CREBBP | increase |
| EP300 | increase |
| KAT2A | increase |
| KAT2B | increase |
| KMT2A | increase |
| SMARCA2 | increase |
| SP100 | increase |
| SP110 | increase |
| TRIM66 | decrease |
| ZMYND8 | decrease |
| ZMYND11 | decrease |
and/or
- the level of expression of at least one bromodomain containing protein selected from the group comprising:
| **Protein** | **Change in expression determining resistance** |
|---|---|
| ATPase family, AAA domain containing 2 | decrease |
| bromodomain adjacent to zinc finger domain, 1A | decrease |
| bromodomain adjacent to zinc finger domain, 1B | increase |
| bromodomain adjacent to zinc finger domain, 2A | decrease |
| bromodomain PHD finger transcription factor | increase |
| bromodomain containing 2 | decrease |
| bromodomain containing 8 | increase |
| cat eye syndrome chromosome region, candidate 2 | increase |
| CREB binding protein | decrease |
| lysine (K)-specific methyltransferase 2A | increase |
| polybromo 1 | increase |
| pleckstrin homology domain interacting protein | increase |
| SWI/SNF related, matrix associated, actin dependent regulator of chromatin, subfamily a, member 4 | increase |
| SP100 nuclear antigen | increase |
| TAF1 RNA polymerase II, TATA box binding protein (TBP)-associated factor, 250kDa | increase |
| tripartite motif containing 28 | decrease |
| tripartite motif containing 33 | increase |
and/or
- the mutations involving the non-synonymous change in amino acid sequence of at least one further bromodomain containing protein selected from the group comprising:
| | **Amino acid change determining resistance** | | **Mutation in parental vs resistant cell lines** | |
|---|---|---|---|---|
| | **Position** | **Reference** | **Parental** | **Resistant** |
| ATAD2 | 365 | Serine | Serine | Phenylalanine |
| ATAD2B | 207 | Glutamine | Arginine | Glutamine |
| BAZ2A | 1 | Methionine | Isoleucine | Methionine |
| BAZ2A | 650 | Glycine | Glycine | Alanine |
| KAT2A | 781 | Arginine | Arginine | Proline |
| SMARCA2 | 855 | Arginine | Glutamine | Arginine |
| TRIM24 | 478 | Proline | Leucine | Proline |
| TRIM24 | 512 | Proline | Leucine | Proline |
| TRIM33 | 286 | Leucine | Leucine | Proline |
| TRIM66 | 630 | Leucine | Valine | Leucine |
| TRIM66 | 324 | Histidine | Arginine | Histidine |
| TRIM66 | 466 | Histidine | Histidine | Arginine |

3. The method according to claim 2, wherein the level of expression of a combination of at least three, four, five, six, seven, eight, nine or ten bromodomain containing genes and optionally also the level of expression of a combination of at least two, three, four, five, six, seven, eight, nine or ten bromodomain containing proteins is determined.

4. The method according to claim 2, wherein the mutations at given reference position in combination of at least three, four, five, six, seven, eight, nine or ten bromodomain containing genes is determined.

5. The method according to any one of the preceding claims, wherein the cancer cells are derived from a cancer selected from carcinomas, sarcomas, melanomas, lymphomas, and leukemia.

## Patentansprüche

1. Verfahren zur Vorhersage der Sensitivität eines Patienten, der an einer Krebserkrankung an einer DNA-Methylierungsinhibitor-Therapie leidet, umfassend die Bestimmung von in vitro in Krebszellen, die vom Patienten genommen wurden, und Vergleich mit Werten für Stammtypen von Zellen
- der Grad der Expression des Bromodomain enthaltenden Gens ASH1L, wobei die Resistenz bestimmenden Expressionsänderung eine Erhöhung ist,
und / oder
- die Mutationen, die die nicht synonyme Veränderung der Aminosäuresequenz von Bromodomain enthaltendem Protein ASH1L beinhalten:
| | **Aminosäureänderung Bestimmung der Resistenz** | | **Mutation in parentalen vs resistenten Zelllinien** | |
|---|---|---|---|---|
| | **Position** | **Referenz** | **Parental** | **Resistent** |
| ASH1L | 1429 | Alanin | Alanin | Valin |

2. Verfahren zur Vorhersage der Sensitivität eines Patienten, der an einer Krebserkrankung an einer DNA-Methylierungsinhibitor-Therapie leidet nach Anspruch 1, Die ferner die Bestimmung von in vitro in Krebszellen, die von dem Patienten genommen wurden, und Vergleich mit Werten für Stammtypen von Zellen umfasst
- der Expressionsgrad von mindestens einem weiteren Bromodomain enthaltenden Gen, ausgewählt aus der Gruppe umfassend:
| **Gen** | **Resistenz bestimmenden Expressionsänderung** |
|---|---|
| ATAD2 | Verminderung |
| BAZ1B | Verminderung |
| BAZ2A | Erhöhung |
| BAZ2B | Verminderung |
| BRD1 | Verminderung |
| BRD3 | Verminderung |
| BRD7 | Verminderung |
| BRD8 | Verminderung |
| BRWD1 | Erhöhung |
| CECR2 | Erhöhung |
| CREBBP | Erhöhung |
| EP300 | Erhöhung |
| KAT2A | Erhöhung |
| KAT2B | Erhöhung |
| KMT2A | Erhöhung |
| SMARCA2 | Erhöhung |
| SP100 | Erhöhung |
| SP110 | Erhöhung |
| TRIM66 | Verminderung |
| ZMYND8 | Verminderung |
| ZMYND11 | Verminderung |
und / oder
- der Expressionsgrad von mindestens einem Bromodomain enthaltenden Protein, ausgewählt aus der Gruppe umfassend:
| **Protein** | **Resistenz bestimmenden Expressionsänderung** |
|---|---|
| ATPase-Familie, AAA-Domain enthaltend 2 | Verminderung |
| Bromodomain neben Zinkfinger Domain, 1A | Verminderung |
| Bromodomain neben Zinkfinger Domain, 1B | Erhöhung |
| Bromodomain neben Zinkfinger Domain, 2A | Verminderung |
| Bromodomain PHD Fingertranskriptionsfaktor | Erhöhung |
| Bromodomain enthaltend 2 | Verminderung |
| Bromodomain enthaltend 8 | Erhöhung |
| Katzenaugen-Syndrom Chromosomen-Region, Kandidat 2 | Erhöhung |
| CREB-bindendes Protein | Verminderung |
| Lysin (K)-spezifische Methyltransferase 2A | Erhöhung |
| Polybrom 1 | Erhöhung |
| Pleckstrin-Homologie-Domäne wechselwirkendes Protein | Erhöhung |
| SWI / SNF verwandte, Matrix assoziierte, aktinabhängige Regulator von Chromatin, Unterfamilie a, Mitglied 4 | Erhöhung |
| SP100-Kernantigen | Erhöhung |
| TAF1-RNA-Polymerase II, TATA-Box-Bindungsprotein (TBP) -assoziierter Faktor, 250kDa | Erhöhung |
| Tripartite Motiv enthaltend 28 | Verminderung |
| Tripartite Motiv enthaltend 33 | Erhöhung |
und / oder
- die Mutationen, die die nicht synonyme Veränderung der Aminosäuresequenz von mindestens einem weiteren Bromodomain enthaltenden Protein einschließen, ausgewählt aus der Gruppe umfassend:
| | **Aminosäureänderung Bestimmung der Resistenz** | | **Mutation in parentalen vs resistenten Zelllinien** | |
|---|---|---|---|---|
| | **Position** | **Referenz** | **Parental** | **Resistent** |
| ATAD2 | 365 | Serin | Serin | Phenylalanin |
| ATAD2B | 207 | Glutamin | Arginin | Glutamin |
| BAZ2A | 1 | Methionin | Isoleucin | Methionin |
| BAZ2A | 650 | Glycin | Glycin | Alanin |
| KAT2A | 781 | Arginin | Arginin | Prolin |
| SMARCA2 | 855 | Arginin | Glutamin | Arginin |
| TRIM24 | 478 | Prolin | Leucin | Prolin |
| TRIM24 | 512 | Prolin | Leucin | Prolin |
| TRIM33 | 286 | Leucin | Leucin | Prolin |
| TRIM66 | 630 | Leucin | Valin | Leucin |
| TRIM66 | 324 | Histidin | Arginin | Histidin |
| TRIM66 | 466 | Histidin | Histidin | Arginin |

3. Verfahren nach Anspruch 2, wobei der Expressionsgrad einer Kombination von mindestens drei, vier, fünf, sechs, sieben, acht, neun oder zehn Bromodomain enthaltenden Genen und gegebenenfalls auch der Expressionsgrad einer Kombination von mindestens zwei, drei, vier, fünf, sechs, sieben, acht, neun oder zehn Bromodomain enthaltenden Proteine wird bestimmt.

4. Verfahren nach Anspruch 2, wobei die Mutationen an einer gegebenen Referenzposition in Kombination von mindestens drei, vier, fünf, sechs, sieben, acht, neun oder zehn Bromodomain enthaltenden Genen bestimmt werden.

5. Verfahren nach einem der vorhergehenden Ansprüche, wobei die Krebszellen aus einem Krebs gewonnen werden, ausgewählt aus Karzinomen, Sarkomen, Melanomen, Lymphomen und Leukämie.

## Revendications

1. Procédé de prédiction de la sensibilité d'un patient souffrant d'une maladie du cancer à un traitement par inhibiteur de la méthylation de l'ADN, qui comprend la détermination *in vitro* dans les cellules cancéreuses prélevées chez le patient et la comparaison avec les valeurs pour les cellules du type parental
- du niveau d'expression du gène ASH1L, dans lequel la variation du niveau d'expression déterminant la résistance est l'augmentation,
et / ou
- les mutations impliquant le changement non synonyme de la séquence d'acides aminés de la protéine ASH1L:
| | **Changement d'acide aminé qui détermine la résistance** | | **Mutation dans les lignées cellulaires parentales vs. résistantes** | |
|---|---|---|---|---|
| | **Position** | **Référence** | **Parentale** | **Résistante** |
| ASH1L | 1429 | Alanine | Alanine | Valine |

2. Procédé de prédiction de la sensibilité d'un patient souffrant d'une maladie cancéreuse à un traitement par inhibiteur de la méthylation d'ADN selon la revendication 1, qui comprend en outre la détermination *in vitro* dans des cellules cancéreuses prélevées chez le patient et la comparaison avec des valeurs pour les cellules parentales
- du niveau d'expression d'au moins un autre gène contenant du bromodomaine choisi dans le groupe comprenant:
| **Gène** | **Changement d'expression qui détermine la résistance** |
|---|---|
| ATAD2 | diminution |
| BAZ1B | diminution |
| BAZ2A | augmentation |
| BAZ2B | diminution |
| BRD1 | diminution |
| BRD3 | diminution |
| BRD7 | diminution |
| BRD8 | diminution |
| BRWD1 | augmentation |
| CECR2 | augmentation |
| CREBBP | augmentation |
| EP300 | augmentation |
| KAT2A | augmentation |
| KAT2B | augmentation |
| KMT2A | augmentation |
| SMARCA2 | augmentation |
| SP100 | augmentation |
| SP110 | augmentation |
| TRIM66 | diminution |
| ZMYND8 | diminution |
| ZMYND11 | diminution |
et / ou
- du niveau d'expression d'au moins une protéine contenant du bromomomaine choisie dans le groupe comprenant:
| **Protéine** | **Changement d'expression qui détermine la résistance** |
|---|---|
| Famille ATPase, domaine AAA contenant 2 | diminution |
| Bromodomain adjacent au domaine du doigt de zinc, 1A | diminution |
| Bromodomain adjacent au domaine du doigt de zinc, 1B | augmentation |
| Bromodomain adjacent au domaine du doigt de zinc, 2A | diminution |
| Bromodomain PHD facteur de transcription des doigts | augmentation |
| Bromodomain contenant 2 | diminution |
| Bromodomain contenant 8 | augmentation |
| Région du chromosome du syndrome des yeux de chat, candidat 2 | augmentation |
| Protéine de liaison CREB | diminution |
| Lysine (K) - méthyltransférase spécifique 2A | augmentation |
| Polybromo 1 | augmentation |
| Protéine d'interaction de domaine d'homologie de pleckstrin | augmentation |
| SWI / SNF associé, associé à la matrice, régulateur dépendant de l'actine de la chromatine, sous-famille a, membre 4 | augmentation |
| Antigène nucléaire SP100 | augmentation |
| TAF1 ARN polymérase II, protéine de liaison à la boîte TATA (TBP) - facteur associé, 250kDa | augmentation |
| tripartite motif contenant 28 | diminution |
| tripartite motif contenant 33 | augmentation |
et / ou
- les mutations impliquant le changement non synonyme dans la séquence d'acides aminés d'au moins une autre protéine contenant du bromodomane choisie dans le groupe comprenant:
| | **Changement d'acide aminé qui détermine la résistance** | | **Mutation in parental vs resistant cell lines** | |
|---|---|---|---|---|
| | **Position** | **Référence** | **Parentale** | **Résistante** |
| ATAD2 | 365 | Serine | Serine | Phenylalanine |
| ATAD2B | 207 | Glutamine | Arginine | Glutamine |
| BAZ2A | 1 | Methionine | Isoleucine | Methionine |
| BAZ2A | 650 | Glycine | Glycine | Alanine |
| KAT2A | 781 | Arginine | Arginine | Proline |
| SMARCA2 | 855 | Arginine | Glutamine | Arginine |
| TRIM24 | 478 | Proline | Leucine | Proline |
| TRIM24 | 512 | Proline | Leucine | Proline |
| TRIM33 | 286 | Leucine | Leucine | Proline |
| TRIM66 | 630 | Leucine | Valine | Leucine |
| TRIM66 | 324 | Histidine | Arginine | Histidine |
| TRIM66 | 466 | Histidine | Histidine | Arginine |

3. Procédé selon la revendication 2, dans lequel le niveau d'expression d'une combinaison d'au moins trois, quatre, cinq, six, sept, huit, neuf ou dix gènes contenant du bromomomaine et éventuellement aussi le niveau d'expression d'une combinaison d'au moins deux, trois, quatre, cinq, six, sept, huit, neuf ou dix protéines contenant du bromodomiane sont déterminées.

4. Procédé selon la revendication 2, dans lequel les mutations à la position de référence donnée en combinaison avec au moins trois, quatre, cinq, six, sept, huit, neuf ou dix gènes contenant du bromomomaine sont déterminées.

5. Procédé selon l'une quelconque des revendications précédentes, dans lequel les cellules cancéreuses dérivent d'un cancer choisi parmi les carcinomes, les sarcomes, les mélanomes, les lymphomes et la leucémie.
